# EUROPEAN PATENT SPECIFICATION

(11) **EP 2 514 829 B1**
(45) Date of publication and mention of the grant of the patent: **02.03.2016**
(21) Application number: 12161815.1
(22) Date of filing: 28.03.2012
(51) Int. Cl.: G01N 33/66, C12Q 1/04

(54) **Detection of bacteria in biological fluids**
Erkennung von Bakterien in biologischen Flüssigkeiten
Détection de bactéries dans des fluides biologiques

(30) Priority: 21.04.2011 US 201113091446
(43) Date of publication of application: 24.10.2012
(73) Proprietor: Pall Corporation, Port Washington, NY 11050 (US)
(72) Inventor: Kwan, Cyndi Leslie Chen, Redondo Beach, CA 90278 (US)
(74) Representative: Hoeger, Stellrecht & Partner Patentanwälte mbB

(56) References cited:
- WO-A2-01/32828
- Y. Suzuki et al.: "Preliminary evaluation of optical glucose sensing in red cellconcentrations using near-infrared diffuse-reflectancespectroscopy", Journal of Biomedical Optics, vol. 17, no. 1 1 February 2012 (2012-02-01), XP55026925, SPIE international Bellingham, Washington USA DOI: 10.1117/1.JBO.17.1.017004] Retrieved from the Internet: URL:http://scitation.aip.org/getpdf/servle t/GetPDFServlet?filetype=pdf&id=JBOPFO0000 17000001017004000001&idtype=cvips&doi=10.1 117/1.JBO.17.1.017004&prog=normal [retrieved on 2012-05-10]
- J. ENG: "Effect of sodium polyanethol sulfonate in blood cultures", JOURNAL OF CLINICAL MICROBIOLOGY, AMERICAN SOCIETY FOR MICROBIOLOGY, US, vol. 1, no. 2, 1 January 1975 (1975-01-01) , pages 119-123, XP002490326, Chicago IL USA ISSN: 0095-1137

## Description

### BACKGROUND OF THE INVENTION

Blood is conventionally processed, e.g., separated into components, to provide a variety of valuable products such as transfusion products. Blood components or products such as buffy coat and platelets may be pooled during processing, e.g., 4-6 units of platelet concentrate can be pooled before administration as a transfusion product. Additionally, blood components processed in a closed system (e.g., without exposing the components to the outside environment) can be stored before administration. For example, red blood cells can be stored for several weeks, and platelets can be stored for several days (e.g., 5 days according to current U.S. practice).

Stored and/or non-stored components can include undesirable material such as bacteria. Bacteria can contaminate the blood or blood component during blood collection (including blood sampling) and/or storage. One source of bacterial contamination may be the blood donor's skin, which may contain one or more varieties of bacteria. Since swabbing the donor's skin (e.g., with alcohol) prior to venipuncture may be inadequate to assure sterility, the bacteria may pass into the blood collection container, and the bacteria may reproduce while the blood or blood component is stored. Additionally, phlebotomy needles may cut a disc of skin when the phlebotomy needle is inserted into the donor, allowing the bacteria-containing skin plug to pass with the blood into the blood collection container.

Other sources of contamination include the donor's blood, the environment (including the air, and the equipment in the environment), and the phlebotomist. Contamination can occur while the unit of blood is being donated and/or while samples of blood are being obtained.

Since some blood components (e.g., platelets) are typically stored at ambient temperatures, the problem of contamination may be magnified, as many species of bacteria reproduce more rapidly at ambient temperatures.

Contaminated blood products, especially bacterially contaminated blood products, pose a potential health risk to those who come into contact with, or receive, these products. For example, the administration of transfusion products with bacterial contamination can have adverse affects on the recipient, and the administration of platelets with massive levels of bacterial contamination is implicated in a number of cases of severe morbidity or death each year in the U.S..

Some existing techniques for detecting bacteria are labor- and time-intensive and may require expensive equipment. Some of the techniques may allow limited sampling, provide inaccurate results, and/or fail to detect certain species of bacteria. Additionally, the techniques may introduce contamination from the environment into the samples.

The present invention provides for ameliorating at least some of the disadvantages of the prior art. These and other advantages of the present invention will be apparent from the description as set forth below.
WO 01/32828 A2 discloses a method and an apparatus for testing a biological fluid for the presence of bacteria. The fluids to be tested have to be depleted from blood cells in order to avoid distortion of the glucose level measurements over time.
J. Suzuki et al. describe in their article "Preliminary evaluation of optical glucose sensing in red cell concentrations using near-infrared diffuse-reflectance spectroscope", Journal of Biomedical Optics, vol 17, No. 1, pages 17004-1 to 17004-7, a way to measure glucose level in transfusion blood in a non-destructive manner.

### BRIEF SUMMARY OF THE INVENTION

In an embodiment, a method for detecting bacteria in biological fluids such as blood and blood products is provided as defined in claim 1, the method comprising placing a biological fluid possibly containing bacteria in contact with a detergent for reducing the respiration of blood cells, and a bacterial growth promoter, and measuring and/or detecting, over a period of time, the level of glucose in a container containing the biological fluid, the detergent, and the bacterial growth promoter.

Another embodiment of the invention provides a system for detecting bacteria in biological fluids such as blood and blood products as defined in claim 6, the system comprising a glucose reading and/or measuring device, and a biological fluid sampling device comprising a container suitable for holding a biological fluid, the container comprising an access port, and containing a detergent for reducing the respiration of blood cells, and a bacterial growth promoter.

Since the respiration of the blood cells (that use glucose as a substrate for their metabolism) is reduced in the presence of the detergent, and since bacteria utilize glucose during their metabolic cycles (aerobic bacteria utilize glucose during glycolysis, anaerobic bacteria utilize glucose during fermentation), if bacteria are present in the biological fluid, bacterial growth will be promoted in the presence of the growth promoter, and the level of glucose will decrease over time. Thus, change in the level of glucose is used as a surrogate marker for bacterial detection.

### BRIEF DESCRIPTION OF THE SEVERAL VIEWS OF THE DRAWING(S)

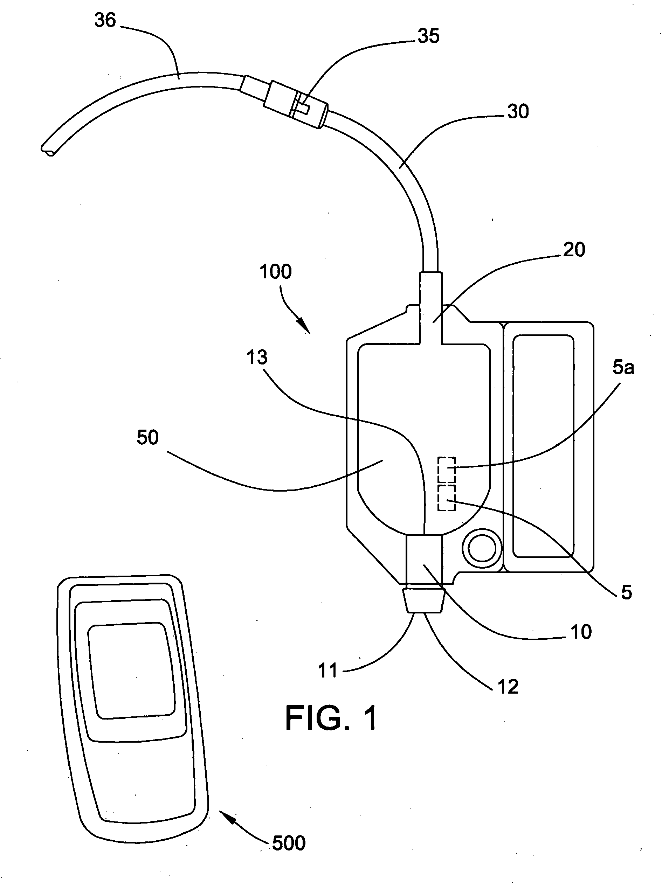

Figure 1 shows an embodiment of a bacteria detection system according to the invention, comprising a biological fluid sampling device comprising a container suitable for holding a biological fluid, the container comprising a glucose sampling port, and containing a detergent for reducing the respiration of blood cells, and a bacterial growth promoter, and a glucose reading and/or glucose measuring device.

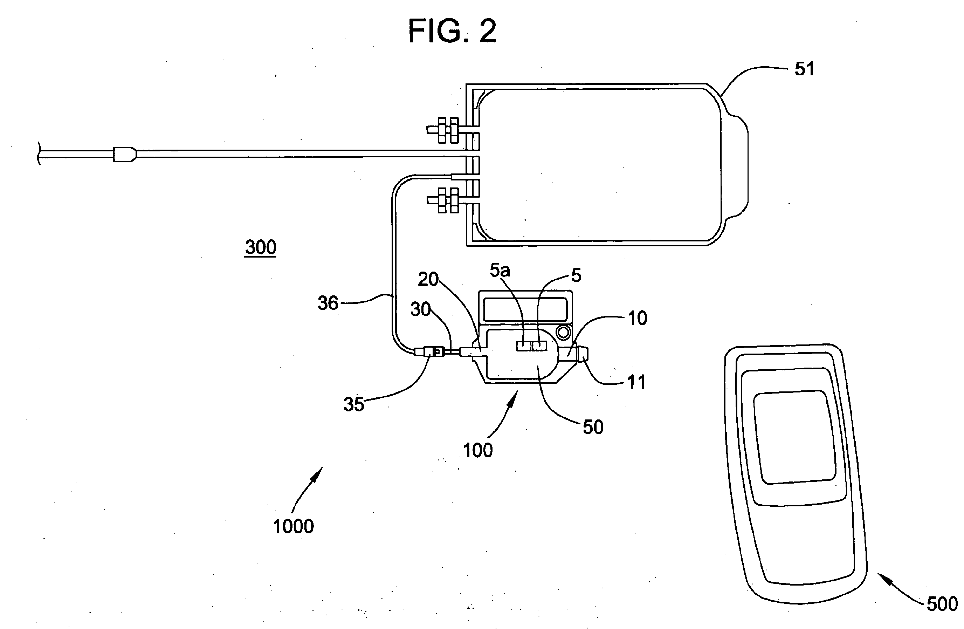

Figure 2 shows another embodiment of a bacteria detection system according to the invention, comprising a glucose reading and/or glucose measuring device, and a biological fluid processing system, comprising a plurality of biological fluid containers, and a biological sampling device as shown in Figure 1.

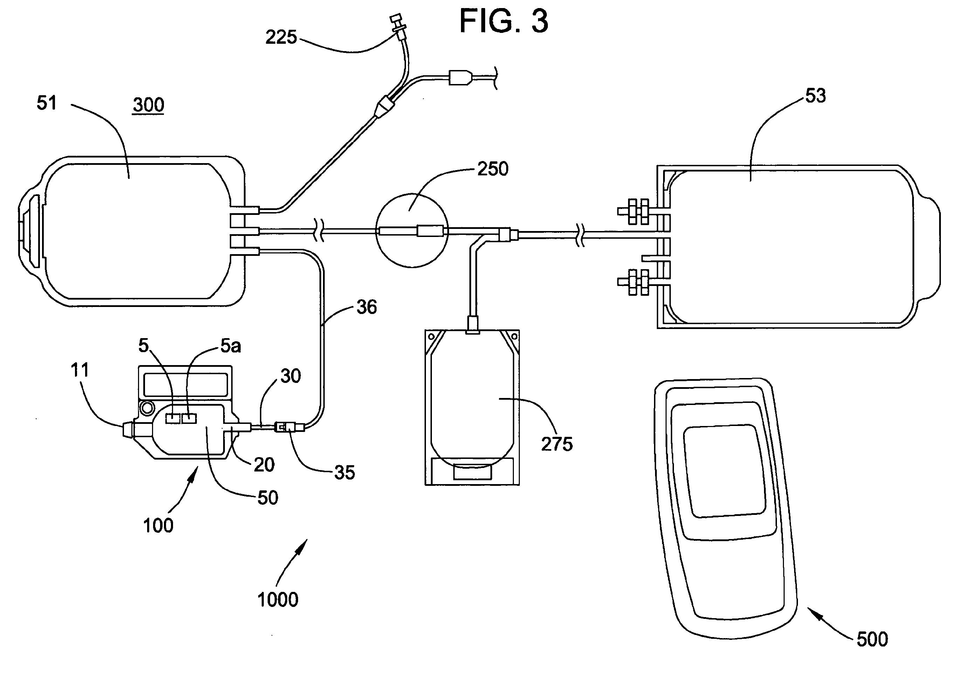

Figure 3 shows another embodiment of a bacteria detection system according to the invention, comprising a glucose reading and/or glucose measuring device, and a biological fluid processing system, comprising a plurality of biological fluid containers, a vent, a leukocyte depletion filter, a vent pouch, and a biological sampling device as shown in Figure 1.

### DETAILED DESCRIPTION OF THE INVENTION

Advantageously, the invention not only allows detection of aerobic bacteria, but also detection of obligate anaerobic bacteria, and detection of facultative anaerobic bacteria not using oxygen for their metabolism.

The inventive method for detecting bacteria in a biological fluid comprises (a) placing a biological fluid, the biological fluid possibly containing bacteria, in a container, the container including. (i) a detergent for reducing the respiration of blood cells, and (ii) a bacterial growth promoter; and, (b) measuring and/or detecting the level of glucose in the biological fluid in the container over a period of time, wherein a decrease in the level of glucose over the period of time indicates the presence of bacteria.

The method further comprises measuring and/or detecting a first level of glucose in the container, and, within about 4 to about 15 hours of measuring and/or detecting the first level, measuring and/or detecting a second level of glucose in the container.

According to the invention, processed biological fluid is placed in the container within about 24 hours or less of originally obtaining biological fluid from a subject.

Additionally, an embodiment of the method comprises collecting a biological fluid from a subject, and, within about 24 hours or less of collecting the biological fluid, passing a portion of biological fluid into the container where the portion of biological fluid is placed in contact with the detergent, and the bacterial growth promoter, and, after a suitable period of time, measuring and/or_ detecting the level of glucose in the container over a period of time. Illustratively, a unit of blood can be collected, and processed to separate the blood into one or more components, including, for example, PC. Within about 24 hours of collecting the blood, a portion of the separated PC can be passed into the container where the portion of PC is placed in contact with the detergent and the bacterial growth promoter, and, after a suitable period of time, measuring and/or detecting the level of glucose in the container over a period of time.

Embodiments of the method can include placing the detergent and the bacterial growth promoter in the container before placing the biological fluid in the container, e.g., the method can comprise placing the biological fluid in a sampling device container, the container comprising a glucose sampling port, and containing a detergent for reducing the respiration of blood cells, and a bacterial growth promoter.

In preferred embodiments, the biological fluid comprises a platelet-containing fluid, such as platelet-rich-plasma, or platelet concentrate; a red blood cell containing fluid, such as whole blood or packed red blood cells; cord blood, stem cell-containing fluid, or a cell culture. In some embodiments, the biological fluid comprises a leukocyte-depleted fluid.

In another embodiment, a system for detecting bacteria in biological fluid is provided, comprising (a) a biological fluid sampling device comprising a container suitable for containing a biological fluid, the container comprising an access port (preferably, a glucose sampling port), the container containing (i) an effective amount of a detergent for reducing the respiration of blood cells, and (ii) an effective amount of a bacterial growth promoter; the system further comprising (b) a glucose reading device and/or a glucose measuring device, the device further comprising a glucose sampling device (such as, for example, a reagent pad and/or a test strip), wherein the reading and/or measuring device is suitable for, over a period of time, measuring and/or detecting a first level of glucose in the biological fluid, and a second level of glucose in the biological fluid, wherein a decrease in the level of glucose over the period of time indicates the presence of bacteria.

Typically, the biological fluid sampling device container suitable for containing the biological fluid is a flexible container.

In a preferred embodiment of the bacteria detection system, the system comprises a biological fluid processing system comprising a plurality of containers, for example, a first container for receiving and/or collecting biological fluid (e.g., a "source container"), and at least a second container (e.g., as part of a biological fluid sampling device, that is also part of the system) for receiving a portion of the biological fluid, wherein the second container contains the detergent and bacterial growth promoter, and the glucose reading and/or measuring device, that detects the levels of glucose in the biological fluid over the period of time. The biological fluid processing system typically includes a plurality of conduits for providing fluid communication between the containers, and may include additional elements such as, for example, one or more additional containers (e.g., for an additive solution and/or for containing processed biological fluid components) and/or a filter device (e.g., a leukocyte depletion filter device) and/or one or more vents such as a gas inlet and/or a gas outlet. Typically, the biological fluid processing system also includes one or more fluid flow control devices such as clamps, transfer leg closures, check valves, and/or rotatable valves.

Embodiments of the bacteria detection system and/or the biological fluid processing system preferably comprise a detachable (or an attachable and detachable) biological fluid sampling device. For example, a biological fluid processing system can be produced including the biological fluid sampling device, or the biological fluid sampling device can be attached (e.g., via sterile docking to maintain sterility) to an existing biological fluid processing system (including any commercially available biological fluid processing system such as a blood bag set). A conduit allowing fluid communication between the first (source) container and the second container (the container of the biological fluid sampling device) can be cut (preferably by heat-sealing to maintain the sterility of the contents of the sampling device and the source container) after the fluid has been passed therethrough, and the bacteria can be subsequently detected.

Alternatively, or additionally, the bacteria detection system and/or the biological fluid processing system can include a biological fluid sampling device that is connected (e.g., to the first container) by a tether, preferably a flexible tether such as a plastic cord or cable. Illustratively, the conduit described above can be cut and sealed, and the separate tether keeps the analysis chamber associated with the source container, e.g., until the analysis for bacteria is completed.

Since bacteria can be detected in accordance with the invention, embodiments of the present invention can be suitable for providing blood components that can be stored for longer periods than are currently allowed by the regulations in various countries. For example, due, at least in part, to fears that platelet concentrate (PC) can be contaminated with bacteria, current U.S. practice requires that, when processed in a closed system, individual units of PC and pooled PC be utilized within 5 days. However, since embodiments of the invention allow the detection of contaminated PC, pooled and unpooled PC can be monitored, and if determined to be uncontaminated, can be used after the 5 day limit that is currently required. Illustratively, individual units of PC or pooled PC processed in a closed system can be transfused after, for example, 7 days of storage.

A wide variety of bacteria, including gram-positive, gram-negative, aerobic, and anaerobic bacteria, utilize glucose, and thus, in accordance with embodiments of the invention, a decrease in the glucose level or concentration in the biological fluid over a period of time reflects the presence of bacteria.

Illustratively, embodiments provide for detecting the presence of bacteria, wherein the bacteria present can be one or more of the following: *Staphylococcus epidermidis*, *Staphylococcus aureus*, *Staphylococcus lugdunensis, Serratia marcescens*, *Serratia liquefaciens*, *Yersinia enterocolitica*, *Klebsiella pneumonia*, *Klebsiella oxytoca*, *Escherichia coli, Enterobacter cloacae, Enterobacter aerogenes*, *Pseudomonas aeruginoisa*, *Eubacterium limosum; Salmonella spp*., such as *Salmonella enterica* (formerly *Salmonella choleraesuis*); *Bacillus spp.*, such as *Bacillus cereus; Clostridium perfringens, Propionibacterium acnes*, *Streptococcus agalactiae* (also known as Group B streptococcus or GBS), *Streptococcus bovis* (at least some strains now called *Streptococcus gallolyticus*), *Streptococcus infantarius,* and *Streptococcus mitis.*

Systems and methods according to the present invention are particularly suitable for use by transfusion services, blood centers and/or blood bank personnel.

If desired, once the presence of bacteria is determined in accordance with the invention, further analysis can be carried out by known techniques to identify the particular species of bacteria present.

Each of the components of the invention will now be described in more detail below, wherein like components have like reference numbers.

Preferably, an embodiment of the sampling device comprises a container having at least two ports, and at least one conduit in fluid communication with at least one of the ports.

Figure 1 illustrates a biological fluid sampling device 100 for use in an embodiment of the invention. The illustrated sampling device comprises a sampling device container 50 (e.g., a "sampling container") for receiving a portion of a biological fluid, the container including an access port 10 (e.g., a "glucose sampling port") and a pierceable element 11 (such as an elastomeric diaphragm) sealing one end of the port, wherein the pierceable element includes a puncturable portion 12 (preferably located at or near the center of the element) allowing insertion of, for example, a liquid withdrawal device (such as, for example, a syringe; typically, wherein the withdrawal device further comprises a needle or nozzle attached to the syringe) or a glucose probe, while maintaining a seal; a biological fluid inlet port 20, and a conduit 30 in fluid communication with the port. In some embodiments, a porous membrane 13, preferably, a hydrophobic microporous membrane, is located at or near the other end of the port. For example, after the needle or nozzle attached to the syringe punctures the diaphragm (without penetrating the membrane 13), the plunger of the syringe can be partially withdrawn in the syringe barrel, creating a vacuum and pulling biological fluid from the container and through the membrane into the barrel. Typically, the access port 10 is made of a different material than the container 50.

Preferably, the conduit 30 is sterile dockable, so that the device can be connected to, for example, another conduit and/or a container, via sterile docking, so that a closed system can be provided. In some embodiments, e.g., as shown in Figure 1, the system comprises a flow control device 35, such as a one-way check valve in fluid communication with the conduit, and at least one additional conduit 36 (preferably, a sterile dockable conduit) in fluid communication with the check valve. The sampling device includes a detergent and a bacterial growth promoter, and the illustrated sampling device includes, within the container, two tablets 5, 5a, each comprising a detergent and a bacterial growth promoter. Preferably, as shown in Figure 1, the device also includes a region suitable for applying indicia, e.g., printed or etched thereon, or for receiving a label with the indicia thereon.

If desired, the sampling device could include, e.g., the access port could further comprise, a luer connector (not shown), allowing a liquid withdrawal device such as a syringe to be connected to the sampling device for subsequent withdrawal of liquid from the container.

Figure 1 also shows a diagrammatic glucose reading device and/or glucose measuring device 500.

Typically, and as illustrated in Figure 2, a bacteria detection system 1000 comprises a biological fluid processing system 300 comprising a plurality of containers, for example, a first container 51 for receiving and/or collecting biological fluid, and at least the second container 50 (as part of the biological fluid sampling device 100 shown in Figure 1, which is also part of the system) for receiving a portion of the biological fluid passed from the first container, wherein the second container contains the detergent and bacterial growth promoter, and wherein a glucose reading device and/or a glucose measuring device 500 detects and/or measures the levels of glucose in the biological fluid over the period of time. The illustrated biological fluid processing system includes a plurality of conduits for providing fluid communication between the containers.

In some embodiments, the bacteria detection system comprises a biological fluid processing system comprising a plurality of containers, and a leukocyte depletion filter. For example, in the embodiment illustrated in Figure 3, the bacteria detection system 1000 comprises a biological fluid processing system 300 comprising a plurality of containers, and a leukocyte depletion filter 250. The illustrated biological fluid processing system 300 comprises a first container 51, e.g., for receiving pooled biological fluid (pooling manifold and containers of individual units of PC not shown), and at least the second container 50 (as part of the biological fluid sampling device 100 shown in Figure 1, which is also part of the system) for receiving a portion of the biological fluid passed from the first container, wherein the second container contains the detergent and bacterial growth promoter, and wherein a glucose reading device and/or a glucose measuring device 500 detects and/or measures the levels of glucose in the biological fluid over the period of time. In accordance with the illustrated system, biological fluid is passed from first container 51 through leukocyte depletion filter 250 into third container 53, which can comprise a storage container. The illustrated biological fluid processing system includes a plurality of conduits for providing fluid communication between the containers.

The biological fluid processing system 300 illustrated in Figure 3 further comprises a vent 225 (that can comprise a gas inlet and/or a gas outlet) and a vent pouch 275, for use as is known in the art. For example, pooled PC can be passed into first container 51, and the vent 225 can allow air to pass through the vent and allow PC in the conduit between the vent and the first container to flow into the container. Subsequently, air in the container 51 can be passed through the conduit and the vent, and the conduit is sealed. In those embodiments wherein the system includes a vent pouch 275, after leukocyte depleted biological fluid is passed into third container 53, air in container 53 can be passed into vent pouch, and the conduit leading to container 53 can be sealed.

While Figure 3 illustrates second container 50 (as part of the biological fluid sampling device 100) in fluid communication with first container 51 (allowing sampling before leukocyte depletion), in another embodiment (not shown), second container 50 (as part of the biological fluid sampling device 100) is in fluid communication with third container 53 (e.g., allowing sampling after leukocyte depletion).

The detergent and/or the bacterial grown promoter can be in dry form (e.g., a powder or a tablet) or in liquid form. In either form, further components, ingredients and/or additives can be included. If desired, e.g., wherein the detergent and/or the bacterial grown promoter are in dry form, for example, tablet form, further components, ingredients and/or additives can include, for example, an inert material such as one or more of the following: maltose, mannitol, and a salt such as calcium chloride, e.g., to provide bulk and/or for ease of binding.

The detergent according to the invention significantly reduces the respiration of blood cells (platelets, red blood cells, and leukocytes) but has little or no effect on bacterial respiration. A preferred detergent is sulforiic polyanethol sodium (SPS).

A variety of bacterial growth promoters are suitable for use in the invention. One preferred promoter is trypticase soy broth (TSB). Other suitable promoters include, but are not limited to, broths such as lysogeny broth (LB broth), mannitol broth, M9 minimal media (that can be supplemented with, for example, one or more of the following: glucose, calcium, and magnesium), or other broths, including supplemented broths.

The glucose level is detected in liquid. For example, a small volume of biological fluid can be removed from the sampling device container (via an access (glucose sampling) port, using a liquid withdrawal device, typically, comprising a syringe) and typically placed on a reagent pad or a test strip which is inserted into a glucose reading device and/or glucose measuring device, and this is repeated at least once after a suitable period of time. Alternatively, for example, a probe can be placed in the sampling device container wherein the container is at least partially filled with biological fluid, the probe is placed in the liquid, and a probe reading can be taken, and the probe is removed and a probe (the same or a different probe) is inserted after a suitable period of time, and another probe reading is taken. Alternatively, the probe can remain in the container allowing multiple and/or continuous readings. A decrease in the glucose level over a period of time indicates bacteria are present.

A variety of equipment, devices and/or protocols are suitable for detecting the level or concentration of glucose in the biological fluid. Illustrative suitable devices 500 (including glucose reading devices and glucose measuring devices) are known in the art and include commercially available devices, available from, for example, LifeScan, Inc. (Milpitas, CA; e.g., Glucometer SureStep Flexx Meters, OneTouch^{®} Blood Glucose Meters), Roche Diagnostics Corp. (Indianapolis, IN; e.g., ACCU-CHEK^{®} meters), Abbott Laboratories (Abbott Park, IL; e.g., Optimum Omega^{™}), Nipro Diagnostics, Inc. (Fort Lauderdale, FL, e.g., TrueTrack^{®} products), and Bayer (Terrytown, NY; e.g., the 1265 Rapidlab^{®} Blood Gas Analyzer).

Glucose reading devices and glucose measuring devices can be set up to provide, for example, "pass" and "fail" results, e.g., wherein a glucose threshold is set up, and once a value below that threshold is detected, the unit of biological fluid "fails" and if a value below that threshold is not detected, the unit of biological fluid "passes." Alternatively, or additionally, the glucose reading devices and glucose measuring devices can be set up to measure glucose levels, wherein the levels are correlated with bacterial contamination, or non-contamination.

One or more probes, sensors, or liquid withdrawal devices can be utilized, e.g., placed in or on the container for the biological fluid. In some embodiments, the components, e.g., liquid withdrawal devices, probes and/or sensors, are self-contained and suitable for one-time use. Embodiments of systems according to the invention can include these items pre-assembled and/or pre-attached, e.g., before biological fluid is passed into the container. Alternatively, or additionally, one or more of these items can be assembled, attached, and/or used during or after the passage of biological fluid into the container.

Embodiments of the invention are suitable for a variety of applications, and the biological fluid can be from a number of sources, preferably mammals. The biological fluid can be from a subject such as a human (e.g., a donor providing a unit of blood or a pheresis product) or an animal. In some embodiments, e.g., in some embodiments wherein biological fluid is to be administered as a transfusion product, or the biological fluid is cord blood or comprises stem cells, the method is preferably carried out while maintaining a closed system.

Embodiments of the method can be carried out in any suitable period of time and at any suitable temperature. For example, a biological fluid can be collected from a subject (and, if desired, processed to separate the biological fluid into one or more components) and maintained at, illustratively, room temperature, for a period of up to about 24 hours from collection, before biological fluid is placed in contact with the detergent and bacterial growth promoter and a first level of glucose in the fluid is measured and/or detected. Typically, biological fluid (e.g., blood) is collected and processed to provide separated components, e.g., wherein one separated component is a unit of PC, and the unit of PC (or pooled PC) is maintained in a first container, and a portion of the PC is passed from the first container to a second container, where it is placed in contact with the detergent and bacterial growth promoter, preferably, wherein the second container contains the detergent and the bacterial growth promoter before the biological fluid is passed into the second container. After a suitable period of time, e.g., after at least about 4 hours (for example, a biological fluid such as PC is placed in contact with the detergent and bacterial growth promoter within about 24 hours of collection of blood, and the suitable period of time is an additional 4 hours), the second glucose level is measured and/or detected. Preferably, in those embodiments wherein the biological fluid is a platelet-containing fluid (e.g., pooled or single unit PC), the platelet-containing fluid in contact with the detergent and the bacterial growth promoter is maintained, for this suitable period of time, at a temperature greater than room temperature, e.g., it is maintained at a temperature of about 35 °C.

The level or concentration of glucose in the biological fluid can be measured and/or detected over any suitable period of time. Typically, the period of time is in the range of from about 4 to about 15 hours, more preferably, about 5 to about 13 hours, between measuring and/or detecting the first level, and measuring and/or detecting the second level.

Preferably, in some of those embodiments wherein the sampling device is detached from the source container, e.g., the source container is no longer in fluid communication with the sampling container, the source container can be processed differently than the sampling device. For example, after a conduit interposed between the source container and the sampling device is sealed and cut, the sampling device can be processed in conditions more conducive to rapid microorganism growth (e.g., stored at a higher than ambient temperature, e.g., a temperature of about 35 to about 37° C), and the source container can be processed in a more conventional manner (e.g., stored at an ambient temperature of about 22° C).

The following definitions are used in accordance with the invention.

Biological Fluid. A biological fluid includes any treated or untreated fluid associated with living organisms, particularly blood, including whole blood, warm or cold blood, cord blood, and stored or fresh blood; treated blood, such as blood diluted with at least one physiological solution, including but not limited to saline, nutrient, additive and/or anticoagulant solutions; blood components, such as platelet concentrate (PC), platelet-rich plasma (PRP), platelet-poor plasma (PPP), platelet-free plasma, plasma, fresh frozen plasma (FFP), components obtained from plasma, packed red cells (PRC), transition zone material or buffy coat (BC); blood products derived from blood or a blood component or derived from bone marrow; stem cells; red cells separated from plasma and resuspended in a physiological solution or a cryoprotective fluid; and platelets separated from plasma and resuspended in a physiological solution or a cryoprotective fluid. A biological fluid also includes a cell culture, and a physiological solution comprising a bone marrow aspirate. The biological fluid may have been treated to remove some of the leukocytes before being processed according to the invention. As used herein, blood product or biological fluid refers to the components described above, and to similar blood products or biological fluids obtained by other means and with similar properties.

A "unit" is the quantity of biological fluid from a donor or derived from one unit of whole blood. It may also refer to the quantity drawn during a single donation. Typically, the volume of a unit varies, the amount differing from patient to patient and from donation to donation. Multiple units of some blood components, particularly platelets and buffy coat, may be pooled or combined, typically by combining four or more units.

As used herein, the term "closed" refers to a system that allows the collection and processing (and, if desired, the manipulation, e.g., separation of portions, separation into components, filtration, storage, and preservation) of biological fluid, e.g., donor blood, blood samples, and/or blood components, without the need to compromise the sterile integrity of the system. A closed system can be as originally made, or result from the connection of system components using what are known as "sterile docking" devices. Illustrative sterile docking devices are disclosed in, for example, U.S. Patents 4,507,119, 4,737,214, and 4,913,756.

If desired, embodiments of the invention can include automated tracking and/or automated detection protocols and equipment. For example, one or more containers and the sampling device can include indicia (e.g., bar coding labels) with information such as one or more of the following: the source(s) of the biological fluid, blood type, additive(s) utilized, an indication whether a specified level of the glucose was reached, incubation temperature, and this information can be tracked, combined with the measured or detected results, and provided in whatever format is suitable, e.g., indicated (in machine readable form if desired) on at least one of the sampling device, source container, and the storage container, and/or as a print-out. This information can be added to the electronic records/database of the user or user's institution, e.g., locally or via a web-based version.

The biological fluid sampling container preferably comprises a flexible container, typically made from materials such as those conventionally used in producing blood bags (e.g., collection bags and/or satellite bags). An embodiment of a biological fluid processing system according to the invention typically comprises a plurality of conduits and containers, preferably flexible containers such as blood bags (e.g., collection bags, satellite bags, and/or storage bags), and vent (air or gas) pouches or bags. In one embodiment, a system according to the invention comprises a closed system. A wide variety of suitable containers and conduits are known in the art. For example, blood collection and satellite bags, and conduits, can be made from plasticized polyvinyl chloride. Bags and/or conduits can also be made from, for example, ethylene butyl acrylate copolymer (EBAC) resin, ethylene methyl acrylate copolymer (EMAC) resin, plasticized ultra-high-molecular weight PVC resin, and ethylene vinyl acetate (EVA). The bags and/or conduits can also be formed from, for example, polyolefin, polyurethane, polyester, and polycarbonate.

Suitable access ports, pierceable diaphragms, flow control devices (including clamps, transfer leg closures, check valves, and rotatable valves), and pooling manifolds, are also known in the art and are commercially available.

In those embodiment including a leukocyte depletion filter, a variety of leukocyte depletion filters are suitable for use in the invention, for example, as described in U.S. Patents 4,880,548, 4,925,572, 5,152,905, and 6,074,869. In those embodiments including a vent such as a gas inlet and/or a gas outlet (e.g., comprising a housing and at least one vent element comprising a porous membrane disposed in the housing), a variety of materials are suitable for use as vent elements. Suitable elements, including hydrophilic microporous membranes and hydrophobic porous membranes, and vents, are disclosed in, for example, U.S. Patent Nos. 5,126,054 and 5,451,321. Preferably, when used in accordance with a closed system, the gas inlet and/or gas outlet prevents the passage of bacteria therethrough, e.g., the gas inlet and gas outlet include a vent element having a bacterial blocking pore rating.

The following examples further illustrate the invention but, of course, should not be construed as in any way limiting its scope.

### EXAMPLE 1

This example demonstrates that glucose measurement can be used to detect the presence of aerobic and anaerobic bacteria.

A sampling device 100 as generally shown in Figure 1 is obtained. The sampling device container 50, which has a volume of about 10 mL, contains 2 tablets, each tablet including 1.75 mg SPS, TSB, calcium chloride, and manufacturing processing agents. Flexible tubing 30 and 36 are each about 2 inches in length.

Whole blood is collected into collection sets including anticoagulant, and processed (including filtration) to provide units of leukocyte-depleted platelet concentrate (PC) in individual containers. After 24 hours, the PC units are inoculated at target inoculums of 1-10 CFU/mL or 100-250 CFU/ML, with the following bacteria.

| **Aerobic/Facultative Anaerobic Bacteria** | **ATCC #** | | **Anaerobic Bacteria** | **ATCC#** |
|---|---|---|---|---|
| *Bacillus cereus* | 7064 | | *Clostridium perfringens* | 13124 |
| *Enterobacter cloacae* | 29005 | | *Eubacterium limosum* | 8486 |
| *Escherichia coli* | 25922 | | *Propionibacterium acnes* | 11827 |
| *Klebsiella pneumoniae* | 8045 | | *Staphylococcus lugdunensis* | 49576 |
| *Pseudomonas aeruginosa* | 27853 | | *Streptococci bovis* | 33317 |
| *Salmonella choleraesuis* | 8326 | | *mitis* | 903 |
| *Serracia marcescens* | 43862 | | | |
| *Streptococci a,galactiae* | 12927 | | | |
| *Staphylococcus aureus* | 27217 | | | |
| *Staphylococcus epidermidis* | 49134 | | | |

After sterile docking the container of PC to the sampling device, approximately 5-6 mL of each inoculated unit is placed in a sampling device as generally shown in Figure 1, using non-gas permeable containers for the anaerobic bacteria and gas permeable containers for the aerobic bacteria, and samples are taken to measure the percent oxygen and glucose levels. The respective sampling devices are incubated under anaerobic and aerobic conditions at 35 °C for 18 and 24 hours with agitation, and samples are taken to measure the percent oxygen and glucose levels at 18 and 24 hours. The glucose levels are measured using a Bayer 1265 Rapidlab^{®} Blood Gas Analyzer (Terrytown, NY) operated in accordance with the manufacturer's instructions.

Additionally, samples are taken 2 hours after inoculation, and the percent oxygen and glucose levels are measured.

Units of non-inoculated PC are used as controls.

The glucose levels at sample times 0 hours and 2 hours for inoculated units are in the range from about 12 to 28 mmol/L, and the glucose levels at sample times 18 hours and 24 hours are about 0 to about 3 mmol/L.

The glucose levels at sample times 0 hours and 2 hours for the controls are in the range from about 12 to 28 mmol/L, and the glucose levels remain at approximately those ranges at sample times 18 hours and 24 hours.

This example demonstrates that the glucose level decreases over time in the inoculated units, reflecting the growth of aerobic and anaerobic bacteria.

### EXAMPLE 2

This example demonstrates that glucose measurement can be used to detect the presence of aerobic and anaerobic bacteria over varying sample device incubation times of 6-24 hours.

Whole blood is collected into collection sets including anticoagulant, and processed (including filtration) to provide units of leukocyte-depleted platelet concentrate (PC) in individual containers. After 24 hours, the PC units are inoculated at target inoculums of 1-10 CFU/mL with the following bacteria.

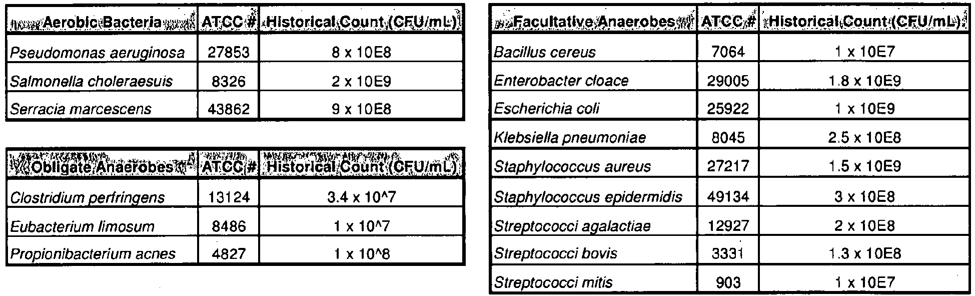

After an additional 24 hours, the containers of PC are sterile docked to sampling devices.

After sterile docking, approximately 5-6 mL of each inoculated unit is placed in a sampling device as generally shown in Figure 1 (including 2 tablets, each tablet including SPS, TSB, calcium chloride, and manufacturing processing agents), using non-gas permeable containers for the anaerobic bacteria and gas permeable containers for the aerobic bacteria, and samples are taken to measure the percent oxygen and glucose levels. The respective sampling devices are incubated under anaerobic and aerobic conditions at 35 °C for 6, 9, 12, and 24 hours with agitation, and samples are taken to measure the pH, percent oxygen.and glucose levels at 6, 9, 12, and 24 hours. The glucose levels are measured using a Bayer 1265 Rapidlab^{®} Blood Gas Analyzer (Terrytown, NY) operated in accordance with the manufacturer's instructions.

Units of non-inoculated PC are used as controls.

The glucose levels for the PC (inoculated and controls) when placed in the sampling devices are about 26 mmol/L. The glucose levels of the inoculated units in the sampling devices at sample times 6 hours, 9 hours, and 12 hours are about 13 mmol/L, 4.5 mmol/L and 4.2 mmol/L, respectively, and the glucose levels for the controls at these sample times remain about 26 mmol/L.

This example demonstrates that the glucose level measurably decreases in 6, 9, and 12 hours of incubation in a sampling device, reflecting the growth of aerobic and anaerobic bacteria.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A method for detecting bacteria in a biological fluid, the method comprising:
(a) obtaining a biological fluid from a subject, the biological fluid possibly containing bacteria, and, within 24 hours or less of obtaining the biological fluid from the subject, passing a portion of the biological fluid into a container, wherein the container includes (i) a detergent for reducing the respiration of blood cells, wherein said detergent comprises sulfonic polyanethol sodium (SPS), and (ii) a bacterial growth promoter; wherein the portion of biological fluid is placed in contact with the detergent and the bacterial growth promoter;
(b) measuring and/or detecting a first level of glucose in the biological fluid in the container over a period of time, and
(c) within 4 to 15 hours of measuring and/or detecting the first level, measuring and/or detecting a second level of glucose in the container, wherein a decrease in the level of glucose from the first level to the second level indicates the presence of bacteria.

2. The method of claim 1, wherein the biological fluid comprises a platelet-containing fluid.

3. The method of claim 1, wherein the biological fluid comprises a stem cell-containing fluid.

4. The method of claim 1, wherein the biological fluid comprises a cell culture.

5. The method of any one of claims 1-4, wherein the biological fluid comprises a leukocyte-depleted fluid.

6. A system for detecting bacteria in biological fluid, comprising:
(a) a biological fluid sampling device comprising a container suitable for containing a biological fluid of a subject, the biological fluid possibly containing bacteria, the container comprising a glucose access port, wherein the container contains (i) an effective amount of a detergent for reducing the respiration of blood cells, wherein said detergent comprises sulfonic polyanethol sodium (SPS), and (ii) an effective amount of a bacterial growth promoter; and,
(b)a glucose measuring device and/or glucose reading device, wherein the device is suitable for, within 24 hours or less of obtaining the biological fluid from the subject, passing a portion of the biological fluid into the container, wherein the portion of biological fluid is placed in contact with the detergent and the bacterial growth promoter, measuring or detecting a first level of glucose in the container, and, within 4 to 15 hours of measuring and/or detecting the first level, measuring and/or detecting a second level of glucose in the container, wherein a decrease in the level of glucose from the first level to the second level indicates the presence of bacteria.

## Patentansprüche

1. Verfahren zum Detektieren von Bakterien in einem biologischen Fluid, das Verfahren umfassend:
(a) Gewinnen eines biologischen Fluids von einem Subjekt, wobei das biologische Fluid möglicherweise Bakterien enthält, und Einleiten eines Teils des biologischen Fluids in einen Behälter innerhalb von 24 Stunden oder weniger nach Gewinnung des biologischen Fluids von dem Subjekt, wobei der Behälter aufweist: (i) ein Detergens zum Vermindern der Atmung von Blutzellen, wobei das Detergens sulfonsaures Polyanetholnatrium (SPS) umfasst, und (ii) einen Promotor für bakterielles Wachstum; wobei der Teil des biologischen Fluids mit dem Detergens und dem Promotor für bakterielles Wachstum in Kontakt gebracht wird;
(b) Messen und/oder Detektieren eines ersten Glucosespiegels in dem biologischen Fluid in dem Behälter über eine Zeitspanne und
(c) Messen und/oder Detektieren eines zweiten Glucosespiegels in dem Behälter innerhalb von 4 bis 15 Stunden nach Messen und/oder Detektieren des ersten Spiegels, wobei ein Absinken des Glucosespiegels von dem ersten Spiegel auf den zweiten Spiegel die Gegenwart von Bakterien anzeigt.

2. Verfahren nach Anspruch 1, wobei das biologische Fluid ein Blutplättchen enthaltendes Fluid umfasst.

3. Verfahren nach Anspruch 1, wobei das biologische Fluid ein Stammzellen enthaltendes Fluid umfasst.

4. Verfahren nach Anspruch 1, wobei das biologische Fluid eine Zellkultur umfasst.

5. Verfahren nach einem der Ansprüche 1-4, wobei das biologische Fluid ein an Leukozyten verarmtes Fluid umfasst.

6. System zum Detektieren von Bakterien in einem biologischen Fluid, umfassend:
(a) eine Vorrichtung zur Probenahme eines biologischen Fluids, umfassend einen Behälter, welcher dazu geeignet ist, ein biologisches Fluid von einem Subjekt aufzunehmen, wobei das biologische Fluid möglicherweise Bakterien enthält, wobei der Behälter einen Glucose-Zugangsport umfasst, wobei der Behälter enthält: (i) eine wirksame Menge eines Detergens zum Vermindern der Atmung von Blutzellen, wobei das Detergens sulfonsaures Polyanetholnatrium (SPS) umfasst, und (ii) eine wirksame Menge eines Promotors für bakterielles Wachstum; und
(b) eine Glucose-Messeinrichtung und/oder eine Glucose-Ableseeinrichtung, wobei die Einrichtung dazu geeignet ist, innerhalb von 24 Stunden oder weniger nach Erhalt des biologischen Fluids von dem Subjekt einen Teil des biologischen Fluids in den Behälter einzuleiten, wobei der Teil des biologischen Fluids mit dem Detergens und dem Promotor für bakterielles Wachstum in Kontakt gebracht wird, Messen oder Detektieren eines ersten Glucosespiegels in dem Behälter und Messen und/oder Detektieren eines zweiten Glucosespiegels in dem Behälter innerhalb von 4 bis 15 Stunden nach Messen und/oder Detektieren des ersten Spiegels, wobei ein Absinken des Glucosespiegels von dem ersten Spiegel auf den zweiten Spiegel die Gegenwart von Bakterien anzeigt.

## Revendications

1. Procédé pour détecter des bactéries dans un fluide biologique, le procédé comprenant :
(a) l'obtention d'un fluide biologique à partir d'un sujet, le fluide biologique contenant éventuellement des bactéries, et, dans les 24 heures ou moins suivant l'obtention du fluide biologique à partir du sujet, le passage d'une partie du fluide biologique dans un récipient, où le récipient comprend (i) un détergent pour réduire la respiration des cellules sanguines, où ledit détergent comprend du polyanéthol sulfonate de sodium (SPS), et (ii) un promoteur de croissance bactérienne ; où la partie du fluide biologique est placée en contact avec le détergent et le promoteur de croissance bactérienne ;
(b) la mesure et/ou la détection d'un premier taux de glucose dans le fluide biologique dans le récipient pendant une certaine période, et
(c) dans les 4 à 15 heures suivant la mesure et/ou la détection du premier taux, la mesure et/ou la détection d'un second taux de glucose dans le récipient, où une réduction du taux de glucose entre le premier taux et le second taux indique la présence de bactéries.

2. Procédé selon la revendication 1, dans lequel le fluide biologique comprend un fluide contenant des plaquettes.

3. Procédé selon la revendication 1, dans lequel le fluide biologique comprend un fluide contenant des cellules souches.

4. Procédé selon la revendication 1, dans lequel le fluide biologique comprend une culture cellulaire.

5. Procédé selon l'une quelconque des revendications 1 à 4, dans lequel le fluide biologique comprend un fluide appauvri en leucocytes.

6. Système pour détecter des bactéries dans un fluide biologique, comprenant :
(a) un dispositif de prélèvement de fluide biologique comprenant un récipient approprié pour contenir un fluide biologique d'un sujet, le fluide biologique contenant éventuellement des bactéries, le récipient comprenant un port d'accès au glucose, où le récipient contient (i) une quantité efficace d'un détergent pour réduire la respiration des cellules sanguines, où ledit détergent comprend du polyanéthol sulfonate de sodium (SPS), et (ii) une quantité efficace d'un promoteur de croissance bactérienne ; et,
(b) un dispositif de mesure du glucose et/ou un dispositif de lecture du glucose, où le dispositif est approprié pour, dans les 24 heures ou moins suivant l'obtention du fluide biologique à partir du sujet, passer une partie du fluide biologique dans le récipient, où la partie du fluide biologique est placée en contact avec le détergent et le promoteur de croissance bactérienne, mesurer ou détecter un premier taux de glucose dans le récipient, et, dans les 4 à 15 heures suivant la mesure et/ou la détection du premier taux, mesurer et/ou détecter un second taux de glucose dans le récipient, où une réduction du taux de glucose entre le premier taux et le second taux indique la présence de bactéries.
